# EUROPEAN PATENT APPLICATION

(11) **EP 4 703 476 A1**
(43) Date of publication of application: **04.03.2026**
(21) Application number: 24196492.3
(22) Date of filing: 26.08.2024
(51) Int. Cl.: C12P 7/04, C12N 9/02, C12P 17/06

(54) **PROCESS FOR THE PREPARATION OF OPTICALLY PURE ALKYL DIOLS AND DERIVATIVES THEREOF**

(71) Applicant: Technische Universität Graz, 8010 Graz (AT)
(72) Inventor: Kourist, Robert, 8010 Graz (AT); Spasic, Jelena, 8010 Graz (AT); Malihan-Yap, Lenny, 8010 Graz (AT); van der Pol, Elske, 8010 Graz (AT); Nigl, Andrea, 8010 Graz (AT)
(74) Representative: Schwarz & Partner Patentanwälte GmbH

(57) **Abstract**

The present invention relates to a method for the preparation of a compound of the formula (I), which is enriched in one enantiomer having formula (I): 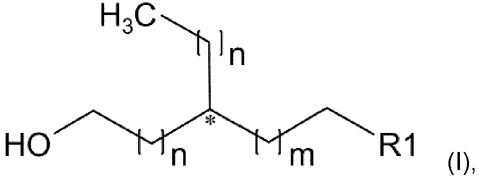 wherein R1 is OH, OCOR2 or COOR3, n is an integer between 0 to 3 and m is an integer between 1 to 10, wherein R2 is H or a C₁ to Cs alkyl group and R3 is H or a C₁ to Cs alkyl group, comprising the step of reacting a compound of formula (II) 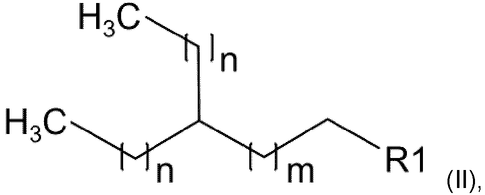 wherein R1, R2, R3, n and m are as defined for formula (I), with an alkane monooxygenase (EC 1.14.15.3), as exemplified for selectively hydroxylating prochiral esters isoamyl acetate, isohexyl acetate and 4-methylpentanoic acid methyl ester.

## Description

### TECHNICAL FIELD

The present invention relates to the field of the production of optically pure alkyl diols and derivatives thereof.

### BACKGROUND ART

To produce drugs containing a chiral active ingredient, it is important to synthesize them as enantiomerically pure as possible. As the different enantiomers of a chiral active ingredient can have very different pharmacological effects, even slight contamination of the chiral active ingredient with the undesirable enantiomer can lead to significant side effects in the patient, which are not acceptable even with strict indications. One possible way to synthesize optically pure compounds is to use enzymes of biological origin, which produce the desired enantiomerically pure product from a prochiral intermediate by enantioselective conversion. A preferred route to synthesis is to use a selective enzyme at a point where the new, enantiomeric product is formed from a prochiral center of the intermediate with high enantioselectivity and regioselectivity. Following this approach, the yield can reach 100%, in comparison to a kinetic resolution which leads up to 50% of the desired enantiomer. Resulting chiral aliphatic alcohols, acids, esters and lactones are a common group of starting compounds for the synthesis of various substances containing a chiral moiety.

Terminal hydroxylation of prochiral dimethyl esters represents a new route towards optically pure diols and their monoesters as well as hydroxycarboxylic acids, their esters and lactones which are present in a broad range of natural products. Among described biocatalysts, bacterial alkane monooxygenases are known as enzymes catalyzing the terminal hydroxylation of linear alkanes (van Beilen, J. B.; et al. Appl Microbiol Biotechnol 2007, 74 (1), 13-21; https://doi.org/10.1007/s00253-006-0748-0; Van Beilen, J. B.; et al. Curr Opin Biotechnol 2005, 16, 308-314; https://doi.org/10.1016/j.copbio.2005.04.005). These membrane-bound enzymes are non-heme monooxygenases displaying these unique features enabling the microorganism to grow on alkanes as a sole carbon and energy source.

### SUMMARY OF THE INVENTION

The present invention relates to a method for the preparation of a compound of the formula (I), which is enriched in one enantiomer having formula (I): 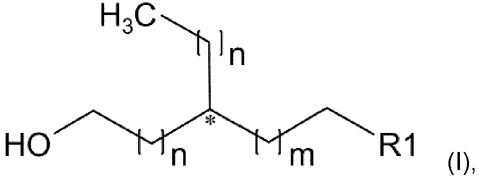 wherein R1 is OH, OCOR2 or COOR3, n is an integer between 0 to 3 and m is an integer between 1 to 10, wherein R2 is H or a C₁ to Cs alkyl group and R3 is H or a C₁ to Cs alkyl group, comprising the step of reacting a compound of formula (II) 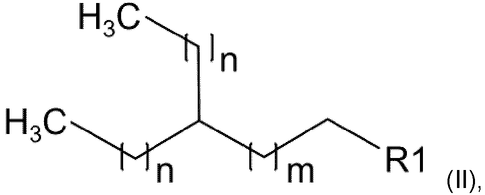 wherein R1, R2, R3, n and m are as defined for formula (I), with an alkane monooxygenase.

Surprisingly, it was found that alkane monooxygenases are able to catalyze the terminal hydroxylation of terminal branched prochiral esters (such as isoamyl acetate). This is an unexpected finding as alkane monooxygenases are known to hydroxylate the linear terminus of the alkanes rather than a sterically demanding branched terminus. In the case of hydroxylation of aliphatic alkyl esters of linear carboxylic acids, for instance, alkane monooxygenases are known to preferentially hydroxylate the acid moiety in the terminal position. Similarly, in the hydroxylation of aliphatic acetate esters of linear organic alcohols, alkane monooxygenases are known to terminally hydroxylate the alcohol moiety. It was also surprising that the hydroxylation of compounds of formula (I) to compounds of formula (II) proceeds in a stereoselective manner allowing the production of enantiomers with up to 100% enantiomeric excess (ee).

### BRIEF DESCRIPTION OF THE FIGURES

Fig. 1 shows a schematic representation of enantioselective hydroxylation of esters (n = 1 - 3) catalyzed by alkane monooxygenase resulting in the production of optically pure alkyl diols, their half-esters, hydroxycarboxylic acids, their esters and lactones.
Fig. 2 shows a reaction scheme of conversion of prochiral alcohol esters catalyzed by alkane monooxygenases. Esters 1a and 2a were tested in whole-cell biotransformations using alkane monooxygenases obtaining hydroxy products and corresponding diols.
Fig. 3A shows gas chromatographic identification of the product after the biocatalytic conversion of isoamyl acetate 2a catalyzed by *E. coli* BL21(DE3) pAlkBFG_ST (black) with an authentic standard produced by lipase-catalyzed transesterification of commercially available racemic 2-methylbutane-1,4-diol (red); BComparison of the mass spectra of the signal at RT = 8.625 minutes.
Fig. 4 shows chiral separation of A racemic 2-methylbutane-1,4-diol 1c and comparison with B hydrolyzed 24h sample of biocatalytic conversion of isoamyl acetate 1a catalyzed by *E. coli* BL21(DE3) pAlkBFG_ST.
Fig. 5 shows A chiral separation of racemic 2-methylpentane-1,5-diol 2c; B chiral separation of hydrolyzed 24h sample of biocatalytic conversion of isohexyl acetate 2a catalyzed by *E. coli* BL21(DE3) pAbAlkBFG_ST.
Fig. 6 shows the enantioselective biocatalytic conversion of acid esters catalyzed by alkane monooxygenase leading to the production of hydroxy acid esters and corresponding lactones. Substrate 3a was tested in whole-cell biotransformation reactions and corresponding lactones were obtained.
Fig. 7 shows A chiral separation of racemic 5-methyltetrahydropyran-2-one 3b; B chiral separation of enantiomer (5R)-tetrahydro-5-methyl-2H-pyran-2-one (R)-3b; C chiral separation of 24h reaction sample from E. *coli* BL21 (DE3) pAlkBFG_ST converting 2mM 4-methylpentanoic acid methyl ester 3a; D comparison of the mass spectra of commercially available 5-methyltetrahydropyran-2-one 3b and the 24h reaction product sample of whole cell biotransformation reaction catalyzed by *E. coli* BL21 (DE3) pAlkBFG_ST using 4-methylpentanoic acid methyl ester 3a as the substrate.

### DESCRIPTION OF EMBODIMENTS

In the method of the present invention the compound of formula (II) is incubated/reacted with an alkane monooxygenase, preferably with an alkane 1-monooxygenase.

The reaction mixture may comprise further components like a buffer. Preferred buffers include KPi, Tris-HCl, TAPS, Bicine, Tricine, TAPSO, HEPES, TES, MOPS, PIPES, Cacodylate, or MES. The buffer maintains the reaction mixture preferably at a pH between 6.5 to 8.5, more preferably between pH 7.3 and 7.7, even more preferably between about 7.4 and 7.5.

According to a preferred embodiment of the present invention, the alkane monooxygenase is an alkane monooxygenase from bacteria, preferably from a genus selected from the group consisting of Pseudomonas, Marinobacter, Alcanivorax, and Fontimonas, more preferably selected from the group consisting of *Pseudomonas putida, Marinobacter sp., Alcanivorax borkumensis,* and *Fontimonas thermophila.*

According to another preferred embodiment of the present invention, the alkane monooxygenase comprises an amino acid sequence that is at least 80%, preferably at least 85%, more preferably at least 90%, even more preferably at least 95%, even more preferably at least 98%, even more preferably at least 99%, in particular 100%, identical to SEQ ID No. 1, SEQ ID No. 2, SEQ ID No. 3, SEQ ID No. 4, SEQ ID No. 5 or SEQ ID No. 6 or which is encoded by a nucleic acid sequence which is at least 80%, preferably at least 85%, more preferably at least 90%, even more preferably at least 95%, even more preferably at least 98%, even more preferably at least 99%, in particular 100%, identical to SEQ ID No. 7, SEQ ID No. 8, SEQ ID No. 9, SEQ ID No. 10, SEQ ID No. 11 or SEQ ID No. 12

The terms "sequence identity", "identity" and "identical", as used herein, refer to the percentage of identical nucleotide or amino acid matches between at least two nucleotide or amino acid sequences aligned using a standardized algorithm. Such an algorithm can, in a standardized and reproducible manner, insert gaps in the compared sequences to optimize the alignment between two sequences, thus achieving a more meaningful comparison of the two sequences.

The percent identity between sequences may be determined using one or more computer algorithms or programs known in the prior art or described herein. According to the present invention, the Basic Local Alignment Search Tool (BLAST) provided by the National Center for Biotechnology Information (NCBI) (Altschul et al., 1990), is preferably used. The BLAST software series contains several programs, including a tool referred to as "BLAST 2 Sequences", which is used for direct pairwise comparison of two nucleotide or amino acid sequences. "BLAST 2 Sequences" can also be accessed and used interactively via the NCBI World Wide Web page on the Internet. The blastn program (for nucleotide sequences) uses as defaults a word length (W) of 28, an expectation (E) of 0.05, M = 1, N = -2, and a comparison of both strands. For amino acid sequences, the blastp pro-gram uses as defaults a word length of 3 and an expectation (E) of 0.05 and the BLOSUM62 scoring matrix (Henikoff & Henikoff, 1989), alignments (B) of 50, expectation (E) of 0.05, M = 1, N = -2.
SEQ ID No. 1: 
MLEKHRVLDSAPEYVDKKKYLWILSTLWPATPMIGIWLANETGWGIFYGLVLLVWYG
ALPLLDAMFGEDFNNPPEEVVPKLEKERYYRVLTYLTVPMHYAALIVSAWWVGTQP
MSWLEIGALALSLGIVNGLALNTGHELGHKKETFDRWMAKIVLAVVGYGHFFIEHNK
GHHRDVATPMDPATSRMGESIYKFSIREIPGAFIRAWGLEEQRLSRRGQSVWSFDN
 
EILQPMIITVILYAVLLALFGPKMLVFLPIQMAFGWWQLTSANYIEHYGLLRQKMEDG
RYEHQKPHHSWNSNHIVSNLVLFHLQRHSDHHAHPTRSYQSLRDFPGLPALPTGY
PGAFLMAMIPQWFRSVMDPKVVDWAGGDLNKIQIDDSMRETYLKKFGTSSAGHSS
STSAVAS
SEQ ID No. 2: 
MSENVLTESLQRDPGAENYVDRKRHLWILSVLWPATPLIGLYLVAQTGWSIWYGLV
LILWYGAVPLIDAMFGEDFSNPPESAVPRLEQDRYYRVLTYLTVPIHYAALIVSAWW
VSTQPMSVFEFLALALSLGIVNGLALNTGHELGHKKETFDRWMAKLVLAVVGYGHF
FIEHNKGHHRDVATPMDPATSRMGESIYSFSLREIPGAFKRAWDLEEQRLSRCGKS
VWSLENEVLQPMILTAVLYAGLLAFFGPLMLIFLPIQMAFGWWQLTSANYIEHYGLLR
EKMSDGRYERQQPHHSWNSNHIMSNLILFHLQRHSDHHAHPTRSYQSLRDFKDLP
SLPSGYPGMFLAAMFPSWFRSIMDHRVLDWAKGDLDKIQIQPGKREFYVRKFGGT
DSESVDTAASK
SEQ ID No. 3: 
MSENILTEPPRSDADNEGYVDRKRHLWILSVLWAATPIIGLYLVSQTGWSIWYGLVLI
LWYGLVPLIDTMLGEDYSNPPESVVPKLEQDRYYKVLTYLTVPIHYAALIISAWWVST
QPIGVFEFLALALSLGIVNGLALNTGHELGHKKETFDRWMAKLVLAVVGYGHFFIEH
NKGHHRDVATPMDPATSRMGESIYTFSLREIPGAFKRAWGLEEQRLSRCGKSVWS
LDNEVLQPMILTVVLYAALLAFFGPLMLIFLPIQMAFGWWQLTSANYIEHYGLLREKL
PNGRYEHQKPHHSWNSNHVMSNLILFHLQRHSDHHAHPTRSYQSLRDFSDLPTLP
TGYPGMFFVAFFPSWFRSLMDDRVMEWAHGDINKIQIQPGMREFYEQKFGVKGSE
SPDTTVAK
SEQ ID No. 4: 
MNGKSSVLDSAPEYVDKKKYFWILSTFWPATPMIGIWLANETGWGIFYGLVLAVWY
GVLPLLDAMFGEDFNNPPEEVVEKLEKERYYRVLTYLTVPMHYAALIVSAWWVGTQ
SMSWFEIVALALSLGIVNGLALNTGHELGHKKEAFDRWMAKIVLAVVGYGHFFIEHN
KGHHRDVATPMDPATSRMGENIYKFSTREIPGAFRRAWGLEEQRLSRRGQSVWSF
DNEILQPMVITVVLYTLLLAFFGPKMLVFLPIQMAFGWWQLTSANYIEHYGLLREKMA
DGRYEHQKPHHSWNSNHIVSNLVLFHLQRHSDHHAHPTRSYQSLRDFPGLPALPT
GYPGAFLMAMIPQWFRSVMDPKVVNWANGDLSKIQIEDSMRAEYIKKFTHNVGAD
DKRGATAVAS
SEQ ID No. 5: 
MSTPTLDAGTLAWNDGKRYLWLLSPFIPVLGLIGLGLFLYTDIGLFTWSGPLLIYGLIP
LLDWLVGEDRNNPPEAAVAQLENDRYYRAIVYAYLPTQYAVTVLGTWVAVTADLAI
WEYIGLVLSVGAVNGIGINTAHELGHKRENLDRWLAKLTLAPVAYGHFFVEHNRGH
HKNVATPEDPASSKMGESFWAFLPRTVIGSLRSAWAIEKARLQRNKQSVWSLDNE
NLQAWAMTIVLFGALTACLGWPALLFLVLQAAYGASLLEVINYIEHYGLLRQKLPDG
RYERCQPRHSWNSNHIVTNLFLYQLQRHSDHHANPTRRFQALRHFDDSPQLPSGY
ASMLIPAYVPWLWFRLMDPLVARHYGGDLTKANLYPPKRAALLARWHRPRPDARR
ADTQPTDATATPADAAASPGGRYQCTDCGYIYDEAIGCPREGFPPGTPWSQIPDD
WSCPDCAVRDKVDFRKLPAA
SEQ ID No. 6: 
MATSMGSHPGFGADSSGTIAYRDRKRPFWTLSVLWPVSPLIGIYLAHTTGVGAFFW
LTLAVWYLIIPLLDWVLGDDQSNPPEAVVPALESDRYYRILTYLTVPIHYVVLIGSAWY
VSTHYGSMTWYDILGLALSVGIVNGLAINTGHELGHKKTELERWLAKIVLAVVGYGH
FFIEHNKGHHKDVATPEDPASAPFGQSIYRFALREIPGAIIRAWNSEKERLGRLGHSP
WSLQNEVLQPLLITIPLYVGLIAVLGVKIIPFLLIQIVFGWWQLTSANYIEHYGLLRQKL
PDGRYERCQPYHSWNSNHVMSNLILFHLQRHSDHHAHPTRRYQSLRDFPDLPTLP
SGYPLMFALSYFPPLWRAVMDRRVLDVCGRDARKINIDPNQREKIIHKFNLLTG
SEQ ID No. 7: 
ATGCTTGAGAAACACAGAGTTCTGGATTCCGCTCCAGAGTACGTAGATAAAAAG
AAATATCTCTGGATACTATCAACTTTGTGGCCGGCTACTCCGATGATCGGAATCT
GGCTTGCAAATGAAACTGGTTGGGGGATTTTTTATGGGCTGGTATTGCTCGTAT
GGTACGGCGCACTTCCATTGCTTGATGCGATGTTTGGTGAGGACTTTAATAATC
CGCCTGAAGAAGTGGTGCCGAAACTAGAGAAGGAGCGGTACTATCGAGTTTTG
ACATATCTAACAGTTCCTATGCATTACGCTGCATTAATTGTGTCAGCATGGTGGG
TCGGAACTCAGCCAATGTCTTGGCTTGAAATTGGTGCGCTTGCCTTGTCACTGG
GTATCGTGAACGGACTAGCGCTCAATACAGGACACGAACTCGGTCACAAGAAG
GAGACTTTTGATCGTTGGATGGCCAAAATTGTGTTGGCTGTCGTAGGGTACGGT
CACTTCTTTATTGAGCATAATAAGGGTCATCACCGTGATGTCGCTACACCGATG
GATCCTGCAACATCCCGGATGGGAGAAAGCATTTATAAGTTTTCAATCCGTGAG
ATCCCAGGAGCATTTATTCGTGCTTGGGGGCTTGAGGAACAACGCCTTTCGCGC
CGTGGCCAAAGCGTTTGGAGTTTCGATAATGAAATCCTCCAACCAATGATCATC
ACAGTTATTCTTTACGCCGTTCTCCTTGCCTTGTTTGGACCTAAGATGCTGGTGT
 
TCCTGCCGATTCAAATGGCTTTCGGTTGGTGGCAGCTGACCAGTGCGAACTATA
TTGAACATTACGGCTTGCTCCGTCAAAAAATGGAGGACGGTCGATATGAGCATC
AAAAGCCGCACCATTCTTGGAATAGTAATCACATCGTCTCTAATCTAGTGCTGTT
CCACCTTCAGCGGCACTCGGATCACCACGCGCATCCAACACGTTCTTATCAGTC
ACTTCGGGATTTTCCCGGCCTGCCGGCTCTTCCGACGGGTTACCCTGGTGCATT
TTTGATGGCGATGATTCCTCAGTGGTTTAGATCAGTTATGGATCCCAAGGTAGTA
GATTGGGCTGGTGGTGACCTTAATAAGATCCAAATTGATGATTCGATGCGAGAA
ACCTATTTGAAAAAATTTGGCACTAGTAGTGCTGGTCATAGTTCGAGTACCTCTG
CGGTAGCATCGTAG
SEQ ID No. 8: 
ATGTCAGAAAATGTACTAACGGAATCCCTGCAACGCGACCCTGGTGCGGAAAAT
TATGTGGACCGAAAACGCCACCTGTGGATCCTGTCTGTTCTGTGGCCAGCAACA
CCGTTGATAGGCCTTTATCTGGTCGCCCAGACCGGATGGAGCATCTGGTACGG
CTTGGTGTTGATCCTTTGGTACGGCGCAGTTCCGCTCATTGATGCCATGTTTGG
TGAGGATTTTAGCAACCCGCCTGAGTCGGCGGTGCCTCGCTTAGAACAGGATC
GTTACTATCGGGTACTGACCTACCTCACGGTTCCTATTCACTATGCCGCCTTGAT
TGTCAGTGCGTGGTGGGTATCAACGCAGCCAATGAGTGTTTTCGAGTTTCTGGC
ATTGGCGCTGTCCCTGGGGATCGTGAATGGCCTGGCTCTGAACACAGGCCATG
AACTGGGCCATAAAAAAGAAACGTTTGACCGTTGGATGGCGAAACTTGTTCTCG
CCGTGGTAGGCTATGGTCATTTCTTCATCGAACACAATAAGGGGCATCACCGCG
ATGTGGCCACCCCGATGGATCCGGCGACTTCCCGCATGGGCGAAAGCATTTAC
AGCTTCTCACTGCGTGAAATTCCCGGTGCTTTCAAGCGGGCATGGGATTTAGAG
GAACAGCGCCTCAGTCGTTGCGGTAAAAGTGTCTGGAGCCTGGAAAATGAAGTT
CTTCAGCCGATGATTTTAACCGCGGTGCTTTATGCGGGATTACTGGCGTTTTTTG
GCCCGTTAATGCTGATCTTTTTGCCGATTCAAATGGCGTTTGGCTGGTGGCAGC
TGACCAGCGCCAACTACATTGAGCATTACGGACTACTGCGCGAGAAGATGAGC
GATGGTCGTTATGAACGTCAGCAACCGCACCACAGCTGGAACTCAAACCATATT
ATGTCGAACCTGATTCTGTTTCATTTACAGCGACATAGCGACCATCACGCCCATC
CGACCCGCTCCTATCAGTCGCTTCGCGACTTCAAAGACCTGCCGAGCTTACCAT
CGGGCTATCCGGGTATGTTCCTGGCCGCAATGTTTCCCTCATGGTTTAGGTCGA
TTATGGACCACCGCGTGCTGGATTGGGCTAAAGGAGATCTCGATAAGATTCAGA
TCCAACCCGGCAAACGTGAATTCTACGTGCGTAAATTCGGTGGGACTGATTCTG
AAAGCGTCGATACCGCAGCGTCTAAATAA
SEQ ID No. 9: 
ATGAGCGAGAACATTTTAACCGAGCCGCCACGAAGTGATGCGGATAATGAAGGT
TATGTGGATCGCAAACGCCACCTGTGGATTCTTTCTGTGCTGTGGGCAGCTACA
CCAATTATTGGCCTATATCTGGTGTCCCAGACTGGGTGGAGTATCTGGTACGGA
TTAGTCCTGATCTTGTGGTACGGCCTCGTCCCGCTGATCGACACCATGCTGGGC
GAGGATTATTCGAACCCTCCGGAATCGGTTGTTCCTAAGCTTGAACAAGACCGC
TACTACAAAGTTTTAACGTACTTAACCGTTCCGATTCATTATGCGGCGTTGATTAT
AAGTGCGTGGTGGGTGTCTACCCAACCAATAGGGGTATTCGAATTTTTAGCTCT
CGCCCTGAGCCTGGGCATCGTTAATGGACTGGCACTGAACACTGGCCATGAAC
TCGGCCATAAAAAAGAAACCTTTGACCGCTGGATGGCCAAGCTGGTACTGGCC
GTGGTCGGCTATGGTCACTTCTTCATCGAACACAATAAAGGGCACCATCGTGAC
GTGGCAACTCCGATGGACCCGGCTACATCCCGCATGGGCGAATCTATTTATACG
TTTTCACTGCGTGAAATTCCGGGTGCCTTTAAACGGGCGTGGGGCTTGGAAGA
GCAGCGCCTCAGCCGTTGCGGCAAAAGCGTGTGGAGCCTAGATAATGAAGTCT
TACAGCCTATGATTTTGACGGTAGTGCTTTATGCCGCATTGCTGGCGTTTTTTGG
TCCGTTAATGCTGATCTTTTTGCCCATTCAGATGGCGTTCGGCTGGTGGCAGCT
GACCAGCGCCAATTATATTGAACACTACGGTCTGCTGCGTGAAAAGCTGCCGAA
CGGTCGCTACGAGCATCAGAAACCGCATCATTCATGGAACAGCAACCATGTGAT
GTCGAACCTCATCCTGTTTCACCTGCAGCGTCATAGTGATCACCACGCGCATCC
TACCCGCTCGTATCAAAGCCTTAGGGACTTCAGCGATCTTCCCACCCTGCCGAC
GGGCTACCCAGGTATGTTCTTCGTCGCATTCTTTCCCTCCTGGTTTCGTTCACTT
ATGGATGATAGAGTGATGGAGTGGGCGCACGGAGATATTAATAAAATCCAGATT
CAGCCGGGCATGCGTGAATTCTATGAACAAAAATTTGGAGTAAAGGGTTCGGAG
TCACCGGATACGACCGTTGCCAAATAA
SEQ ID No. 10: 
ATGAATGGCAAAAGCAGCGTTCTGGATAGCGCGCCCGAATACGTGGACAAAAA
AAAATATTTTTGGATTCTGTCGACCTTTTGGCCGGCTACTCCGATGATCGGAATT
TGGCTGGCCAACGAGACTGGCTGGGGTATCTTTTATGGTTTAGTCCTAGCAGTG
TGGTATGGGGTACTTCCGCTGCTTGACGCGATGTTCGGTGAGGACTTTAACAAT
CCGCCGGAGGAAGTAGTGGAGAAACTGGAGAAAGAACGTTACTATCGAGTTTTA
ACGTATCTTACCGTTCCAATGCATTACGCAGCACTGATCGTGTCTGCCTGGTGG
GTAGGGACACAGTCAATGTCCTGGTTCGAAATCGTCGCCCTGGCCTTGAGTCTG
GGCATCGTGAATGGCCTAGCGCTGAACACCGGCCACGAACTGGGTCATAAAAA
AGAAGCCTTTGATCGTTGGATGGCGAAAATTGTGCTGGCGGTTGTCGGGTATG
GCCATTTCTTTATTGAGCATAACAAAGGCCATCACCGTGATGTTGCGACCCCGA
TGGACCCTGCGACCTCCCGCATGGGAGAAAATATATATAAATTCTCAACCCGCG
AAATTCCTGGAGCGTTTCGTCGTGCTTGGGGCTTGGAAGAACAGCGCTTATCGC
 
GCCGCGGCCAAAGCGTTTGGAGTTTCGATAATGAGATCCTCCAGCCAATGGTCA
TTACAGTGGTGCTCTACACCCTCCTGCTTGCCTTCTTTGGTCCGAAGATGCTGG
TGTTCCTTCCAATTCAGATGGCCTTCGGGTGGTGGCAGTTAACGAGCGCGAACT
ACATTGAACACTACGGTTTGCTGCGTGAAAAAATGGCCGATGGAAGGTATGAAC
ATCAAAAGCCTCATCACTCGTGGAACAGTAACCACATCGTGTCTAACCTGGTCC
TGTTTCACTTACAGCGCCATTCTGATCACCATGCGCACCCGACGCGCTCCTACC
AGAGCTTACGTGATTTCCCCGGCCTGCCGGCACTCCCGACTGGCTACCCCGGT
GCCTTTTTGATGGCAATGATTCCGCAATGGTTTAGATCGGTGATGGACCCTAAG
GTGGTAAATTGGGCTAATGGCGACCTGAGCAAGATTCAGATTGAAGATTCAATG
CGCGCCGAATATATTAAAAAGTTTACGCATAATGTTGGTGCGGATGATAAACGG
GGCGCAACCGCGGTAGCAAGCTAA
SEQ ID No. 11: 
ATGTCTACGCCGACACTGGATGCAGGAACCTTAGCTTGGAACGACGGGAAACG
TTATTTGTGGTTACTTTCCCCATTCATTCCGGTACTGGGTCTGATCGGTCTTGGT
TTATTTCTTTATACCGATATAGGTTTATTTACCTGGTCTGGACCTCTGCTGATTTA
TGGTTTAATACCGCTGTTGGATTGGCTGGTTGGCGAGGATCGCAATAACCCACC
AGAAGCGGCCGTTGCGCAGTTGGAAAACGATAGATACTACCGTGCTATTGTGTA
TGCGTACCTGCCGACCCAGTATGCGGTGACGGTTCTGGGCACCTGGGTTGCCG
TGACTGCGGACCTGGCGATTTGGGAGTACATTGGCCTAGTCCTGTCAGTAGGC
GCCGTTAACGGCATTGGCATTAATACTGCACACGAACTGGGACATAAACGTGAG
AATCTGGACCGCTGGCTCGCAAAGCTGACCCTCGCACCAGTGGCGTACGGTCA
CTTTTTTGTGGAACACAACCGGGGACATCATAAAAACGTTGCGACACCGGAGGA
TCCGGCCTCAAGCAAAATGGGTGAATCCTTCTGGGCGTTCCTGCCCCGAACGG
TCATTGGCTCATTGCGTAGCGCTTGGGCCATCGAAAAAGCTCGCCTGCAGCGC
AATAAGCAGAGCGTGTGGTCGCTCGATAACGAAAATCTGCAGGCGTGGGCCAT
GACTATCGTCTTATTTGGCGCCCTGACCGCATGTCTCGGGTGGCCAGCGCTGC
TGTTTCTGGTACTACAGGCAGCGTATGGCGCCAGTTTACTTGAGGTGATCAACT
ATATTGAACATTATGGCTTGCTGCGTCAAAAATTGCCGGACGGTCGCTATGAAC
GTTGCCAGCCGCGCCACAGCTGGAATAGCAATCACATTGTCACCAACCTGTTTC
TCTACCAGCTGCAACGTCATTCCGATCATCATGCAAACCCGACTCGCAGATTCC
AAGCCCTGCGACATTTCGATGACAGTCCTCAATTGCCCTCGGGTTACGCCTCGA
TGTTAATCCCAGCGTATGTGCCGTGGCTGTGGTTTCGGCTGATGGACCCGTTG
GTCGCTCGTCATTATGGGGGTGACCTGACCAAAGCAAATCTTTATCCACCTAAA
CGCGCAGCACTTCTGGCCCGCTGGCACCGCCCTCGCCCCGATGCGCGTCGCG
CGGATACGCAGCCGACCGATGCTACGGCAACACCGGCCGATGCGGCTGCTAG
TCCCGGCGGCCGTTACCAATGCACAGACTGCGGTTACATCTATGATGAAGCGAT
 
CGGCTGTCCGCGGGAAGGGTTCCCGCCGGGGACGCCTTGGTCTCAGATCCCG
GACGATTGGAGCTGCCCTGATTGTGCCGTGCGTGACAAGGTAGATTTTAGGAAA
CTCCCGGCGGCCTAA
SEQ ID No. 12:

ATGGCCACTTCAATGGGCTCCCATCCTGGTTTCGGTGCAGACAGCTCGGGAAC
AATTGCGTATAGGGATCGTAAACGCCCGTTCTGGACCCTCAGCGTTCTTTGGCC
CGTAAGTCCGCTGATCGGAATTTACCTTGCGCATACCACTGGCGTGGGTGCCTT
CTTTTGGCTCACGCTGGCGGTTTGGTACTTGATTATTCCGCTCCTGGACTGGGT
TCTTGGTGATGATCAAAGCAACCCACCTGAAGCGGTGGTGCCGGCTTTGGAGT
CGGATCGCTATTACCGTATTCTTACCTATTTAACGGTGCCGATACACTATGTGGT
GCTAATCGGCAGTGCGTGGTATGTCTCGACCCACTACGGGTCTATGACGTGGT
ATGATATACTCGGTCTGGCTCTGAGTGTTGGCATTGTCAATGGTCTTGCAATTAA
CACAGGCCACGAACTGGGCCATAAAAAAACCGAACTGGAACGTTGGCTGGCGA
AGATTGTGTTGGCCGTAGTGGGGTACGGTCACTTTTTTATTGAACATAATAAAGG
CCATCATAAAGATGTTGCTACCCCAGAAGATCCCGCCAGTGCACCATTTGGTCA
GTCAATTTACCGTTTTGCCCTGCGTGAAATCCCTGGCGCGATCATCCGTGCCTG
GAACAGCGAAAAGGAGCGCTTAGGCCGGCTGGGTCATAGCCCGTGGTCTTTAC
AAAATGAAGTGTTACAACCTCTGCTGATCACGATACCGCTATATGTTGGCCTGAT
TGCGGTACTGGGGGTTAAAATTATCCCGTTTCTTCTGATCCAGATTGTATTCGGT
TGGTGGCAACTGACGTCCGCCAATTATATCGAGCATTACGGGCTGCTGCGCCA
GAAGCTGCCGGATGGACGCTACGAACGTTGTCAGCCGTATCACTCATGGAACA
GCAACCACGTCATGTCCAACCTGATCTTGTTCCACTTACAGCGCCATTCCGATC
ATCATGCACATCCGACCCGCCGTTATCAGTCTTTACGAGACTTTCCGGACCTGC
CGACACTCCCCTCGGGGTACCCATTAATGTTCGCGCTGAGCTATTTTCCTCCGC
TGTGGCGCGCTGTCATGGACAGACGCGTGTTGGATGTCTGCGGCCGGGATGCA
CGGAAAATTAATATTGACCCAAACCAGCGAGAGAAAATCATCCACAAATTTAATC
TGTTGACTGGCTAA

One of the most studied alkane monooxygenases is AlkB from *Pseudomonas putida* GPo1 (*P*. *putida*) (UniProt SEQ ID P12691; SEQ ID No. 1). AlkB is a monooxygenase known for selectively catalyzing the terminal hydroxylation of alkanes (C₃-C₁₂), cycloalkanes, thioesters and esters of fatty acids and alcohols with broad chain-length specificity and exclusive terminal C-H bond selectivity. AlkB system also overoxidized its substrates forming not only the ω-alcohol but also the corresponding aldehyde as well as the carboxylic acid. It has turned out that AlkB is particularly suited to catalyze the reaction of the present invention. Hence, it is particularly preferred to use an alkane monooxygenase comprising or consisting of an amino acid sequence which is at least 80% identical to SEQ ID No. 1.

According to a further preferred embodiment of the present invention the alkane monooxygenase comprising the amino acid sequence SEQ ID No. 1 comprises a mutation at position F164 and/or I233.

It turned out that a mutation at position F164 and/or I233 of SEQ ID No. 1 results in an alkane monooxygenase variant which allows to convert a compound of formula (II) in a compound of formula (I) to an even higher enantiomeric excess compared to an alkane monooxygenase comprising or consisting of SEQ ID No. 1.

Position F164 and/or position I233 of SEQ ID No. 1 are preferably substituted with a valine, leucine or isoleucine residue. Particularly preferred mutations are selected from the group consisting of F164L, F164I and I233V. A further preferred variant of SEQ ID No. 1 comprises a mutation of both F164 and I233, more preferably of F164L and I233V.

According to a preferred embodiment of the present invention the C₁ to Cs alkyl group of R2 and/or R3 is a methyl or ethyl group.

According to another preferred embodiment of the present invention the compound of formula (II) is reacted with the alkane monooxygenase in the presence of an electron transfer protein and an enzyme regenerating the electron transfer protein, preferably an electron transfer protein reductase.

Alkane monooxygenase requires electrochemically derived reducing equivalents i.e. electrons, which may be passed indirectly to the monooxygenase by means of a suitable carrier such as an electron transfer protein. Electron transfer proteins may be ferredoxin or rubredoxin, wherein rubredoxin is particularly preferred.

In order to regenerate the electron transfer protein, the reaction mixture may comprise optionally an enzyme regenerating the electron transfer protein. Such regeneration enzymes require in many cases the presence of cofactors like NAD(P)H in the reaction mixture.

According to a preferred embodiment of the present invention, the electron transfer protein is rubredoxin and the enzyme regenerating the electron transfer protein is rubredoxin reductase.

Rubredoxin, an iron-sulphur containing protein, is a particularly preferred electron donor for alkane monooxygenases. Rubredoxin is regenerated with rubredoxin reductase, which transfers electrons from NADH to rubredoxin. Thus, it is particularly preferred to react the compound of formula (II) with the alkane monooxygenase in the presence of rubredoxin as well as rubredoxin reductase.

According to a further preferred embodiment of the present invention, rubredoxin comprises an amino acid sequence that is at least 80%, preferably at least 85%, more preferably at least 90%, even more preferably at least 95%, even more preferably at least 98%, even more preferably at least 99%, in particular 100%, identical to SEQ ID No. 13 or SEQ ID No. 14 or which is encoded by a nucleic acid sequence which is at least 80%, preferably at least 85%, more preferably at least 90%, even more preferably at least 95%, even more preferably at least 98%, even more preferably at least 99%, in particular 100%, identical to SEQ ID No. 15 or SEQ ID No. 16.
SEQ ID No. 13: 
MASYKCPDCNYVYDESAGNVHEGFSPGTPWHLIPEDWCCPDCAVRDKLDFMLIES
GVGEKGVTSTHTSPNLSEVSGTSLTAEAVVAPTSLEKLPSADVKGQDLYKTQPPRS
DAQGGKAYLKWICITCGHIYDEALGDEAEGFTPGTRFEDIPDDWCCPDCGATKEDY
VLYEEK
SEQ ID No. 14: 
MSRYQCPDCQYIYDENKGEPHEGFHPNTSWNDIPKDWACPDCAVRDKVDFIFLAD
SPSKETQLGVNSQLANSESGISDATPTGMAVLAAELVIPLNQENKNEGCAAKTEVLD
QASTPQVVRKSSTRKKMRNK
SEQ ID No. 15: 
ATGGCTAGCTATAAATGCCCGGATTGTAATTATGTTTATGATGAGAGTGCGGGTA
ATGTGCATGAGGGGTTTTCTCCAGGTACGCCTTGGCACCTTATTCCTGAGGATT
GGTGCTGCCCCGATTGCGCCGTTCGAGACAAGCTTGACTTCATGTTAATTGAGA
GCGGCGTAGGTGAAAAGGGCGTCACCTCAACCCATACTTCGCCAAATTTATCCG
AGGTTAGTGGCACAAGTTTAACTGCTGAAGCAGTGGTTGCGCCGACAAGCTTAG
AGAAATTGCCTAGTGCCGACGTTAAAGGCCAAGATCTATATAAAACTCAACCTCC
AAGGTCTGATGCCCAAGGCGGGAAAGCATACTTGAAGTGGATATGTATTACTTG
TGGCCATATATATGATGAGGCGTTGGGCGATGAGGCCGAGGGTTTTACTCCAG
GTACTCGCTTTGAGGATATTCCTGATGACTGGTGCTGTCCGGATTGCGGGGCTA
CGAAAGAAGACTATGTGCTCTACGAGGAAAAGTGA
SEQ ID No. 16:

ATGTCAAGGTACCAGTGTCCAGATTGTCAGTATATCTATGATGAAAATAAGGGG
GAGCCGCACGAAGGTTTCCACCCGAACACCAGCTGGAATGATATCCCCAAAGA

TTGGGCATGCCCGGACTGCGCAGTTCGAGACAAGGTGGACTTTATCTTTCTCGC
GGATTCTCCCTCGAAAGAAACACAGCTAGGGGTGAATAGTCAGCTTGCCAACTC
GGAAAGTGGTATTTCAGATGCTACTCCAACTGGAATGGCAGTTTTGGCCGCAGA
ATTAGTGATCCCACTTAATCAAGAAAATAAAAATGAGGGCTGTGCGGCTAAGACT
GAAGTTCTTGATCAGGCGAGCACCCCACAGGTTGTAAGAAAATCTTCCACAAGG
AAGAAGATGAGAAATAAATAA

According to another preferred embodiment of the present invention, rubredoxin reductase comprises an amino acid sequence that is at least 80%, preferably at least 85%, more preferably at least 90%, even more preferably at least 95%, even more preferably at least 98%, even more preferably at least 99%, in particular 100%, identical to SEQ ID No. 17 or which is encoded by a nucleic acid sequence which is at least 80%, preferably at least 85%, more preferably at least 90%, even more preferably at least 95%, even more preferably at least 98%, even more preferably at least 99%, in particular 100%, identical to SEQ ID No. 18.
SEQ ID No. 17: 
MAIVVVGAGTAGVNAAFWLRQYGYKGEIRIFSRESVAPYQRPPLSKAFLTSEIAESA
VPLKPEGFYTNNNITISLNTPIVSIDVGRKIVSSKDGKEYAYEKLILATPASARRLTCEG
SELSGVCYLRSMEDAKNLRRKLVESASVVVLGGGVIGLEVASAAVGLGKRVTVIEAT
PRVMARVVTPAAANLVRARLEAEGIEFKLNAKLTSIKGRNGHVEQCVLESGEEIQAD
LIVVGIGAIPELELATEAALEVSNGVVVDDQMCTSDTSIYAIGDCAMARNPFWGTMV
RLETIHNAVTHAQIVASSICGTSTPAPTPPRFWSDLKGMALQGLGALKDYDKLVVAIN
NETLELEVLAYKQERLIATETINLPKRQGALAGSIKLPD
SEQ ID No. 18:

ATGGCAATCGTTGTTGTTGGCGCTGGTACAGCTGGAGTAAATGCTGCGTTCTGG
CTTCGTCAATATGGTTATAAAGGGGAAATTAGGATTTTTAGCAGGGAGTCTGTG
GCGCCTTATCAGCGGCCTCCTCTATCCAAGGCTTTTCTGACAAGTGAGATTGCA
GAATCCGCAGTGCCATTAAAGCCAGAAGGTTTTTATACGAATAACAATATTACCA
TTTCGTTAAATACACCGATTGTATCAATCGACGTGGGGCGTAAGATAGTTTCTTC
TAAAGATGGAAAAGAATACGCGTATGAAAAATTGATTCTTGCAACACCTGCTAGC
GCACGTAGGTTAACCTGCGAGGGGTCTGAACTGTCTGGGGTCTGCTATTTACG
CAGTATGGAAGACGCCAAAAATTTACGTAGGAAACTTGTGGAGAGTGCGTCTGT
TGTTGTGTTGGGCGGCGGAGTAATCGGGCTTGAAGTCGCCTCAGCTGCGGTGG
GCTTAGGGAAGAGGGTCACAGTGATAGAAGCCACCCCGCGTGTAATGGCGCGC

GTGGTTACGCCGGCAGCAGCAAACTTAGTCAGAGCCCGCCTGGAGGCTGAAGG
AATTGAGTTCAAGCTGAATGCGAAATTAACGTCTATAAAGGGCAGGAATGGCCA
TGTTGAACAATGCGTACTTGAAAGTGGAGAAGAAATTCAGGCGGATCTGATTGT
AGTTGGAATCGGTGCTATCCCAGAGCTAGAGCTGGCAACTGAGGCGGCCCTTG
AAGTGAGTAATGGTGTTGTGGTCGATGATCAGATGTGTACATCGGATACAAGTA
TATATGCAATCGGCGACTGCGCAATGGCTAGAAATCCTTTTTGGGGAACGATGG
TACGTTTAGAGACAATTCATAATGCGGTTACACACGCTCAAATTGTCGCAAGTAG
CATCTGTGGCACATCAACACCAGCACCAACCCCACCACGGTTCTGGTCTGATCT
TAAAGGGATGGCGCTGCAAGGACTTGGTGCTCTAAAGGACTACGATAAACTCGT
TGTTGCAATTAATAACGAAACTCTTGAACTAGAAGTCCTTGCGTACAAGCAGGAG
CGACTGATTGCAACTGAGACAATAAATTTGCCTAAACGTCAAGGTGCGCTTGCA
GGGAGTATAAAATTACCTGATTAG

The electrons for the hydroxylation reaction catalyzed by the alkane monooxygenase of the present invention can be supplied from an electron transfer protein reductase via an electron transfer protein as mentioned above to the monooxygenase. Particularly suited is NADH-dependent rubredoxin reductase (AlkT) which transfers electrons via rubredoxin (AlkG) to AlkB mentioned above. Hence, it is particularly preferred to use in the method of the present invention an electron transfer protein that comprises or consists of an amino acid sequence being at least 80% identical to SEQ ID No. 13 or SEQ ID No. 14. The electron transfer protein reductase comprises or consists particularly preferred of an amino acid sequence being at least 80% identical to SEQ ID No. 17.

According to a preferred embodiment of the present invention, the alkane monooxygenase is an isolated alkane monooxygenase or comprised in a cell expressing the alkane monooxygenase and optionally the electron transfer protein and/or the electron transfer protein reductase, preferably rubredoxin and/or the rubredoxin reductase, or a lysate of said cell.

The proteins required for the preparation of the enantiomerically pure products of the present invention can be used as isolated and/or purified proteins, comprised (i.e. expressed) in cells or part of a lysate of said cells. It is particularly preferred to provide the proteins used in the method of the present invention in the form of a whole-cell biocatalyst expressing said proteins. The whole-cell biocatalyst may be further modified to increase the yield of the product, for example by deletion or inactivation of enzymes capable of degrading or metabolizing the substrate or product of the inventive reaction method.

In a preferred embodiment of the present invention, the term "cell", as used herein, refers to bacteria, archaea, fungi, algae, and the like. In a preferred embodiment, the cell is a bacterial cell, more preferably one from the group comprising *Pseudomonas, Corynebacterium* and *Escherichia,* most preferably *Escherichia coli.* In another preferred embodiment, the cell is a lower eukaryote, more preferably a fungus from the group comprising *Saccharomyces, Candida, Picchia, Schizosaccharomyces,* and *Yarrowia.*

The present invention is further illustrated in the following example, however, without being restricted thereto.

### EXAMPLE

### Material and methods

### Synthesis of isohexyl acetate 2a

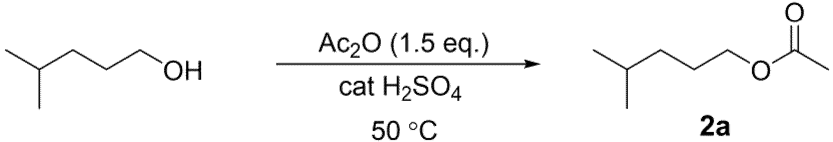

To a 10 mL flame-dried round bottom flask were added 4-methylpentan-1-ol (863 µL, 102 mg, 1.00 mmol, 1 eq.) and acetic anhydride (983µL, 153 mg, 1.50 mmol, 1.5 eq.). While stirring, 2 drops of concentrated H₂SO₄ were added and after 10 min, the mixture was placed in a preheated oil bath maintained at 50 °C. To follow the reaction, a 90-minute sample was analyzed by thin-layer chromatography (cyclohexane:EtOAc (ethyl acetate), 2:1, KMnO₄, R_{f} starting material, 0.45 R_{f} product 0.81). Additionally, the sample was analyzed by GC-MS indicating the reaction went to completion. The reaction mixture was diluted with Et₂O (2x 7.5 mL) and washed with saturated NaHCOs (20 mL, 2x). The organic layer was dried over Na₂SO₄, filtered and concentrated under reduced pressure. The product is very volatile. To obtain a pure product without organic solvents, a huge loss of product is observed. Hence, a low yield is reported. Yield: 80 mg, 0.056 mmol, 8% as a colorless oil.

¹H NMR (300 MHz, CDCl₃) δ 4.04 (t, *J* = 6.8 Hz, 2H), 2.05 (s, 3H), 1.72 - 1.46 (m, 3H), 1.20 (dt, J = 29.5, 14.9 Hz, 2H), 0.89 (d, *J* = 6.6 Hz, 6H).

### Cloning and expression of AlkBFG_ST and homologs

AlkBFG_ST was cloned similarly as previously described into the broad-host vector pCom10_AlkL (Julsing, M. K.; et al. Appl Environ Microbiol 2012, 78 (16), 5724-5733. https:/Idoi.org/10.1128/AEM.00949-12) by excising AlkL resulting in the vector p AlkBFG_ST (van Nuland, Y. M.; et al. Appl Environ Microbiol 2016, 82 (13), 3801-3807. https://doi.org/10.1128/AEM.00822-16; Schrewe, M.; Magnusson, A. O.; Willrodt, C.; et al. Adv Synth Catal 2011, 353 (18), 3485-3495. https://doi.org/10.1002/adsc.201100440). Synthetic DNA-fragments encoding *alkBFG* and *alkT* were ordered from Integrated DNA Technologies. The sequence information was taken from the *P. putida* OCT plasmid alk genes cluster (GenBank entry: AJ245436.1). The fragments were sub-cloned into the pJET1.2 vector using the Thermo Scientific CloneJET PCR Cloning Kit. The *alkBFG* and alkTfragments were inserted into the pCom10 vector backbone via the FastCloning method (Li, C.; et al. BMC Biotechnol 2011, 11 (1), 92. https://doi.org/10.1186/1472-6750-11-92). The correct assembly of pAlkBFG_ST was verified by colony PCR as well as by Sanger Sequencing. The synthetic DNA fragments encoding homologs from *Pseudomonas putida* P1 (GenBank SEQ ID AJ233397), *Marinobacter sp.* (GenBank SEQ ID NYTA01000006 REGION: 105278...106492) and *Alcanivorax borkumensis* (GenBank SEQ ID AB110225) were ordered from Integrated DNA Technologies and were cloned analogously to the method described above.

*E. coli* BL21(DE3) harboring the pAlkBFG_ST plasmid was used to express AlkBFG_ST or corresponding homologs. *E. coli* BL21(DE3) pCom10 (empty vector) was used for the control experiments. *E. coli* strains were either grown in LB or M9 minimal medium (1×M9 salts, containing 2-fold amounts of NH₄Cl 0.5% glucose, 2 mM MgSO₄, 1 ml L⁻¹ US^{Fe} trace elements, 1 mg L⁻¹ thiamine, 5 µg L⁻¹ biotin,) supplemented with 50 µg mL-' kanamycin for the selection. 4 mL of LB medium was inoculated with a single colony and incubated overnight at 37 °C and constant agitation (120 rpm). 200 µL of the overnight culture (ONC) was transferred into 20 mL of fresh M9 minimal media in a 100 mL shake flask and the cell culture was incubated overnight at 30 °C and constant agitation (120 rpm). For the expression, 50 - 200 mL of M9 minimal medium were inoculated with the M9-grown preculture to a starting OD₆₀₀ of 0.15 and grown at 30 °C until an OD₆₀₀ of 0.4 to 0.6. The recombinant gene expression was induced by the addition of 0.05 % (v/v) dicyclopropyl ketone (DCPK). After 4 h of the expression at 30 °C with constant agitation (120 rpm), the cells were harvested by centrifugation (4,400 × g, 4 °C, 15 min).

### Whole-cell biotransformation

After the harvesting, the cells were resuspended in the resting cell buffer (50 mM KPi, pH 7.4, 1 % glucose, 2 mM MgSO₄) to an OD₆₀₀ of 10. The reaction was performed in a 20 mL tightly sealed glass vial with a total volume of 1 mL. The reaction was started by the addition of 2 mM of the substrate (substrate stock of 200 mM in EtOH or DMSO). The cells were incubated for 24 h at 30 °C and 180 rpm. 1mL reaction samples taken at different time points were stored at - 20 °C until further use. Before the extraction, to 250 µL of reaction sample, 25 µL of 2 M HCl was added. Substrate conversion and product formation were analyzed by GC-MS and GC-FID.

### Lipase-catalyzed transesterification of 2-methylbutane-1,4-diol

To obtain the standard of the product after biotransformation of substrate isoamyl acetate 1a, transesterification of a racemic 2-methyl-1,4-butanediol with vinyl acetate using lipase from *Pseudomonas cepacia* (enzyme/substrate ratio 312 U mmol⁻¹) was done accordingly to previously published literature (Ferraboschi, P.; et al. Tetrahedron Asymmetry 1994, 5 (4), 691-698. https://doi.org/10.1016/0957-4166(94)80031-6; Grisenti, P.; et al. Tetrahedron Asymmetry 1993, 4 (5), 997-1006. https://doi.org/10.1016/S0957-4166(00)80144-5). The reaction was performed by mixing 0.48 mmol diol, 0.96 ml chloroform, 0.177 mL vinyl acetate and 3.88 mg lipase in a round-bottom flask maintained at 30 °C in an oil bath. The 24h sample of the reaction was filtered and 10 µL of the reaction was extracted with 990 µl EtOAc by vigorously shaking. The organic phase separation was achieved by centrifugation (16,000 × g, 4 °C, 5 min). The organic phase was dried over Na₂SO₄ and 200 µL of extract was analyzed by GC-MS.

### Analytics

### GC-MS

To confirm the formation of the desired products, analysis by GC-MS was done. The conditions for analytics were established using commercially available compounds. All measurements were performed on a Shimadzu GCMS-QP2010 SE instrument equipped with an AOC-20i/s autosampler and injector unit together with a Zebron ZB-5ms capillary column (30 m × 0.25 mm × 0.25 µm, Phenomenex). The method used for the analysis is presented in Table 1. For the analysis of the whole-cell biotransformation, 25 µL of 2 M HCl was added to 250 µL reaction sample and extracted with 250 µL of EtoAc (1:1, v/v) by vigorous shaking. The organic phase separation was achieved by centrifugation (16,000 × g, 4 °C, 5 min). The organic phase was dried over Na₂SO₄ and 200 µL of extract was directly analyzed by GC-MS.

**Table 1 Parameters of the GC-MS method used to analyze product formation**

| | | |
|---|---|---|
| **Column** | **Name** | **ZB-5ms** |
| | Phase | Phenyl-arylene phase |
| | Dimensions | Thickness: 0.25 µm |
| | | Length: 30.0 m |
| | | Diameter: 0.25 mm |
| | Temperature | 50°C |

| **Temperature** | Profile | Hold: 50°, 3 min |
|---|---|---|
| | | Heating: to 300°C, 30°C min⁻¹ |
| | | Hold: 300°C, 3 min |
| **Injection port** | Volume | 1 µL |
| | Carrier Gas | He |
| | Temperature | 250°C |
| | Split Ratio | 9.1 |
| **FID Detector** | Temperature | Ion source: 250°C |
| | | Interface: 250°C |
| | Total flow | 15.0 mL min⁻¹ |
| | Column flow | 1.19 mL min⁻¹ |
| | Pressure | 67.2 kPa |

### GC-FID

To confirm the formation of enantiomers, analysis by GC-FID equipped with a chiral Hydrodex βTBDAc column was done. All measurements were performed on a Shimadzu Nexis GC-2030 instrument equipped with an AOC-20s Plus autosampler and injector unit together with a Hydrodex β-TBDAc column (50 m × 0.25 mm × 0.25 µm, Hydrodex). The methods used for the analysis are presented in Table 2. For the analysis of the whole-cell biotransformation where hydrolysis was employed, 200 µl of 5M NaOH was added to 800 µL of 24h reaction sample and hydrolyzed for 1h at 40 °C, 700 rpm. The product was extracted with EtoAc, adding 1 mL EtoAc to 1 mL of the reaction sample (1:1, v/v) and extracted by vigorously shaking. The organic phase separation was achieved by centrifugation (16,000 × g, 4 °C, 5 min). The organic phase was dried over Na₂SO₄ and 200 µL of extract was directly analyzed by GC-FID.

**Table 2 Parameters of GC-FID method used to analyze product formation.**

| | | **Compound 1c** | **Compound 2c** | **Compound 3c** |
|---|---|---|---|---|
| **Chiral column** | **Name** | Hydrodex βTBDAc column | | |
| | Phase | HYDRODEX cyclodextrin phase | | |
| | Dimensions | Length: 50 m | | |
| | | Inner diameter: 0.25 mm | | |
| | | Film Thickness: 0.25 µm | | |

| **Temperature** | Profile | Hold: 120 °C, 10 min | Hold: 125°C, 10 min | |
|---|---|---|---|---|
| | | Heating: to 137 °C, 0.5 °C min^{- 1}, Heating: to 220 °C, 10 °C min^{- 1}, hold 2 min | Heating: to 137°C, 0.5°C min⁻¹, Heating: to 220°C, 10°C min⁻¹, hold 2 min | Hold: 90 °C, 3 min |
| | | | | Heating: to 220 °C, 2 °C min⁻¹, hold 3 min |
| **Injection port** | Volume | 1 µL | | |
| | Carrier Gas | N₂ | | |
| | Temperature | 230 °C | | |
| | Split Ratio | 10 | | |
| **FID Detector** | Temperature | 250 °C | | |
| | Air flow rate | 200 mL min⁻¹ | | |
| | H₂ flow rate | 32 mL min⁻¹ | | |

### Results

### Esters of alcohols

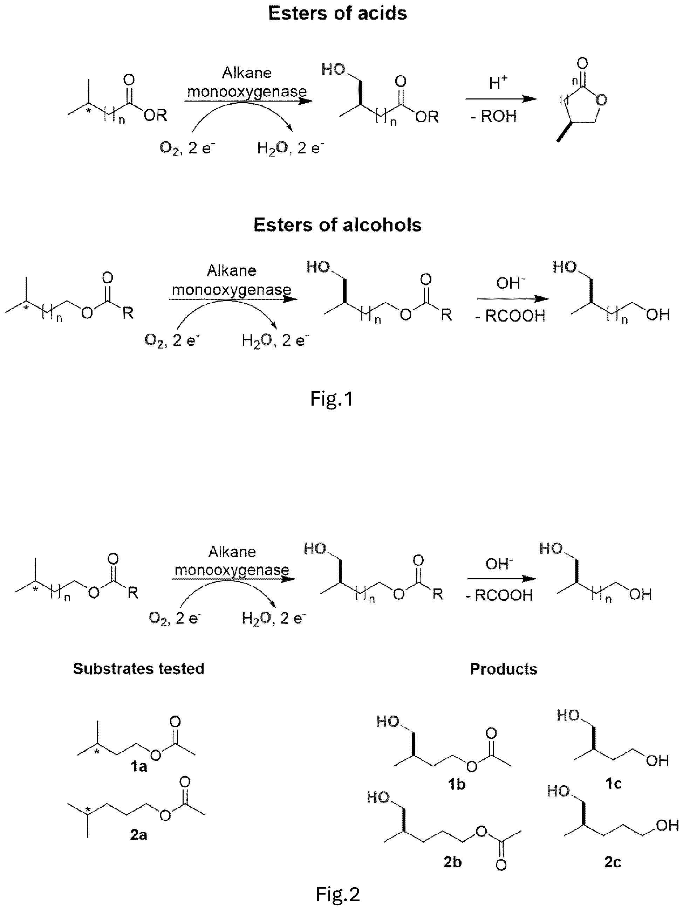

To perform enantioselective terminal hydroxylation, a whole-cell biotransformation with *E*. *coli* BL21(DE3) cells expressing the pAlkBFG_ST system was used to selectively hydroxylate prochiral ester isoamyl acetate 1a and isohexyl acetate 2a (Figure 2).

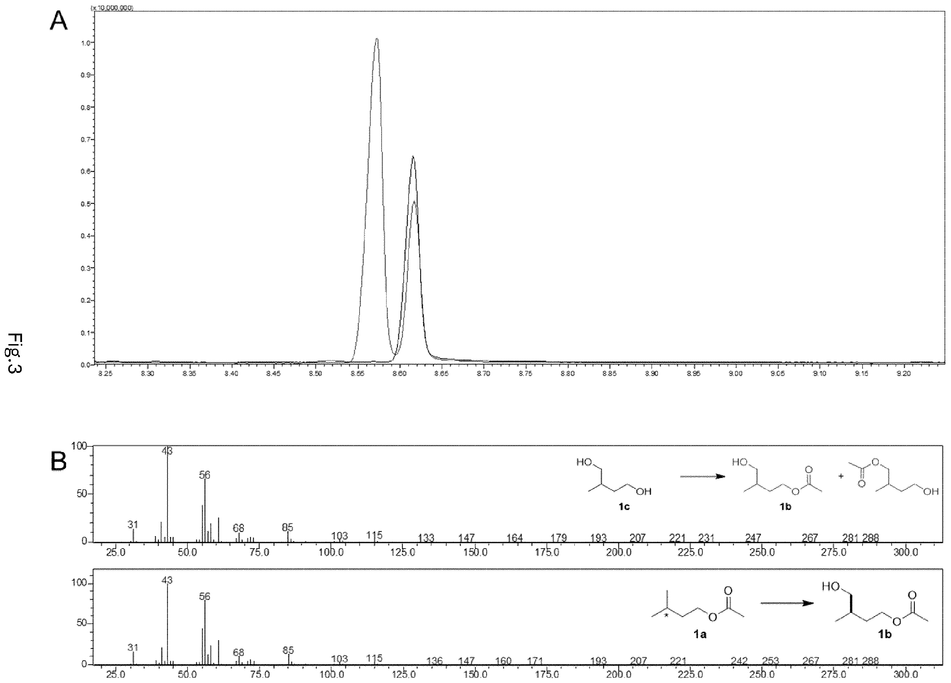

After 4h of expression, the cells were harvested and resuspended in a resting cell buffer (KPi, pH 7.4, 1 % glucose, 2 mM MgSO₄) to an OD₆₀₀ of 10. The reaction was started by adding 2 mM of substrate isoamyl acetate 1a (200 mM stock in EtOH) and performed in a total volume of 1 mL at 30 °C and 180 rpm. The samples for GC-analysis were taken directly at the beginning of the reaction and after 24 hours the of reaction time. The reaction mixture was extracted with EtOAc and dried over Na₂SO₄. The GC-MS analysis of the 24 h sample revealed a newly formed product identified as the compound 4-acetoxy-2-methylbutane-1,4-diol 1b. The mixed products of lipase-catalyzed transesterification of the racemic 2-methylbutane-1,4-diol 1c, 4-acetoxy-1-methylbutane-1,4-diol 1b and 1-acetoxy-1-methylbutane-1,4-diol, were used as the reference compounds. The identity of the reaction product was confirmed by comparing the retention time and mass spectrogram of the product formed by AlkB from 1a and the lipase transesterification product 1b (Figure 3). The described monoester 4-acetoxy-2-methylbutane-1,4-diol 1b would be rather difficult to produce using other methods, as chemical synthesis starting from the diol would always result in a mixture of both half esters and the diesters. The downstream separation of these monoesters would be very complex. No product formation could be observed in biotransformation with *E*. *coli* cells containing the empty expression pCom10 vector.

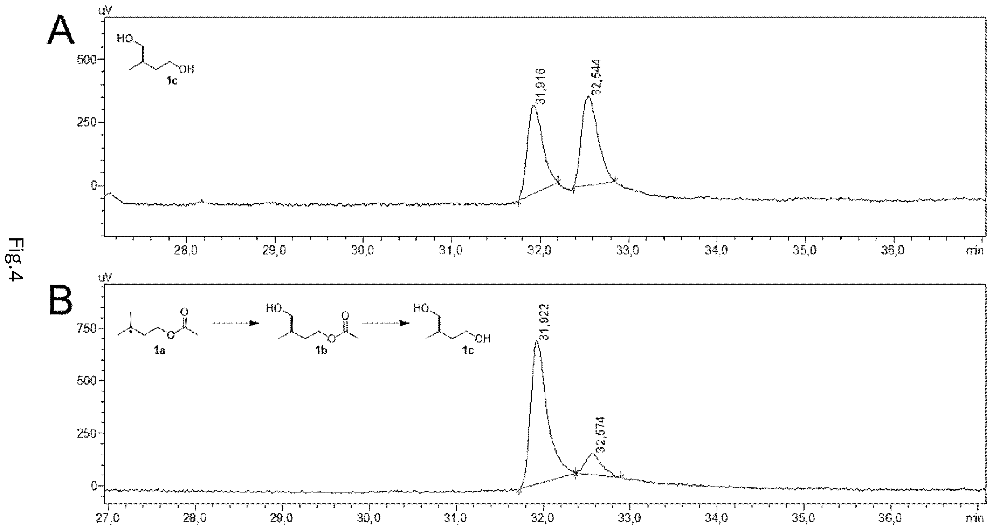

To analyze the optical purity of the enzymatically formed 4-acetoxy-2-methylbutane-1,4-diol 1b, the compound was converted to 2-methylbutane-1,4-diol 1c via base-catalyzed saponification (1M NaOH, 40 °C, 700rpm, 1h). The retention time of both enantiomers in chiral GC-analysis was determined with commercially available racemic 2-methylbutane-1,4-diol 1c. An enantiomeric excess of 86% ee was determined (Figure 4). Different variants of AlkB were tested (F164L, I233V, F164I and a double mutant of F164L and I233V), reaching up to 88% ee for the variant I233V.

Homologous alkane monooxygenases originating from *Marinobacter*sp. (UniProt SEQ ID A0A7G2RNR8), Alcanivorax borkumensis (UniProt SEQ ID Q0VKZ3) and *Pseudomonas* putida P1 (UniProt SEQ ID Q9WWW6) were tested in whole-cell biotransformation of 2mM isoamyl acetate 1a reactions resulting in up to 84% ee.

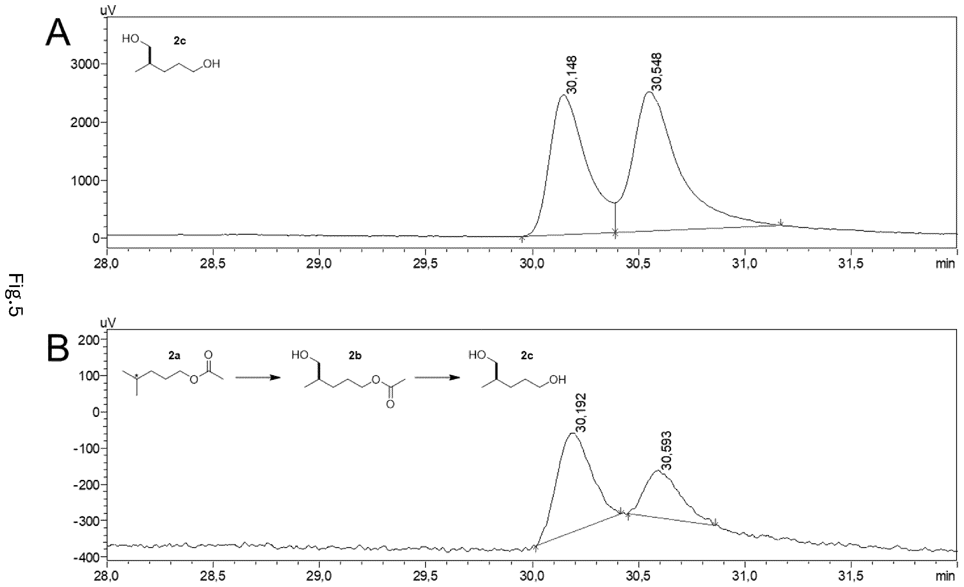

To further exemplify the application of alkane monooxygenases for the asymmetric conversion of prochiral esters, AlkB and homologs were tested for the conversion of prochiral isohexyl acetate 2a. The reaction was performed analogously to the abovementioned reaction setup. The 24-hour reaction sample was analyzed for the optical purity of the enzymatically formed 1,5-pentanediol, 2-methyl-, 5-acetate 2b. The compound was converted to 2-methylpentane-1,5-diol 2c via base-catalyzed saponification (1M NaOH, 40 °C, 700rpm, 1h) and the retention time was compared with the racemic 2-methylpentane-1,5-diol 2c on the GC-FID equipped with chiral Hydrodex βTBDAc column. The highest enantiomeric excess up to 41% *ee* was determined for a homolog from *Alcanivorax borkumensis* (AbAlkB) (Figure 5).

### Esters of acids

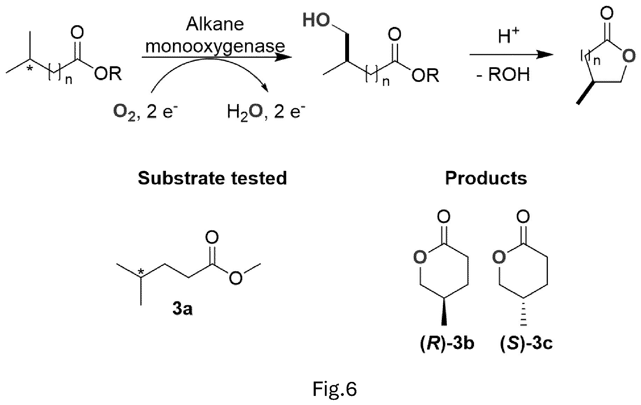

The reaction was performed analogously to the whole-cell biotransformation of alcohol esters 1a and 2a using 2 mM of 4-methylpentanoic acid methyl ester 3a as a substrate (Figure 6) and *E*. *coli* BL21(DE3) cells expressing pAlkBFG_ST. The resulting ω-hydroxy acid esters can be cyclized to the corresponding lactones via acid-catalyzed cyclization while retaining the stereo-conformation (Figure 6). These lactones could be also produced by enzymatic Baeyer-Villiger oxidation of the corresponding ketones, however, this produces a mixture of the 'normal' and 'abnormal' lactone as the oxygen can be inserted at both sides of the carbonyl group, usually leading to the formation of four lactones.

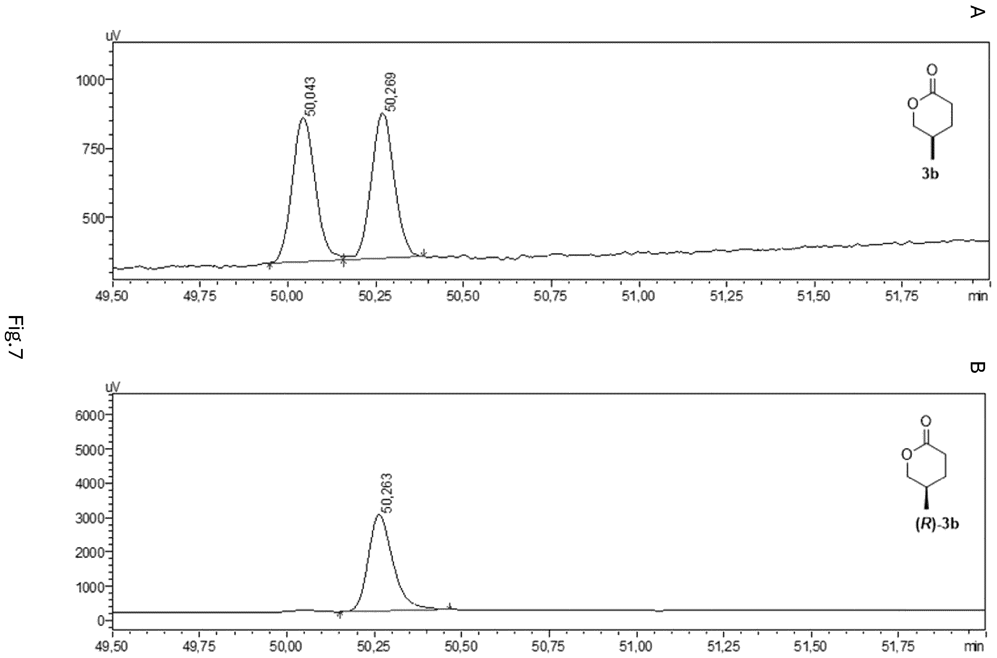

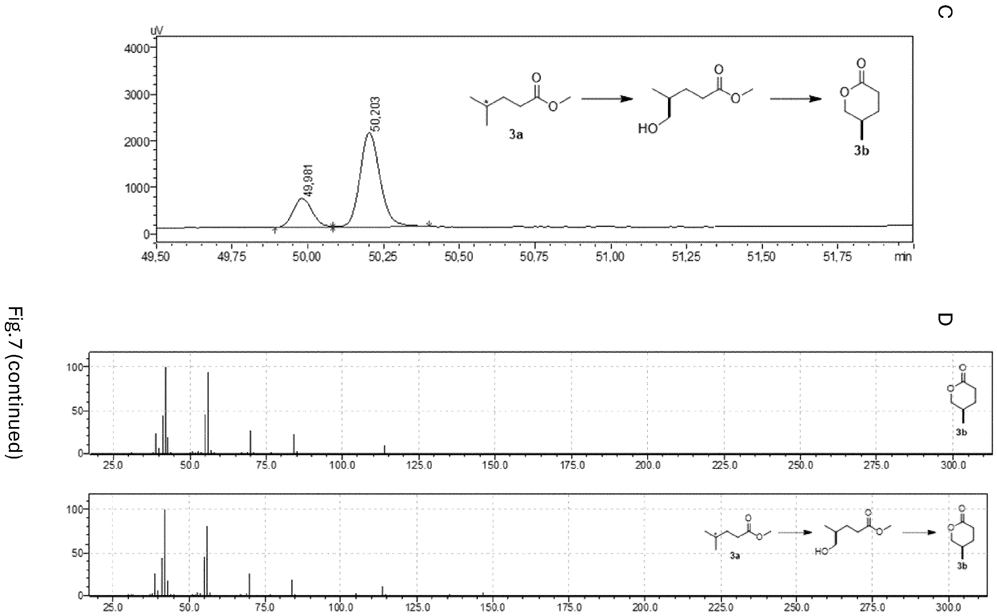

The whole-cell biotransformation of prochiral 2 mM 4-methylpentanoic acid methyl ester 3a using *E*. *coli* BL21 (DE3) pAlkBFG_ST was done following the same protocol as for the prochiral alcohol esters. The GC-MS analysis of the 24 h sample indicated the formation of a hydroxy product and a lactone (Figure 7). The preference toward the formation of (R)-enantiomer was confirmed by chiral GC-FID analysis with a clear overlap between a peak of 24 h reaction sample and (5R)-tetrahydro-5-methyl-2H-pyran-2-one (R)-3b as the standard of the lactone. The enantioselectivity of AlkB resulted in 54% ee toward (R)-3b. No product formation could be observed when *E*. *coli* cells containing the empty pCom10 vector were used as a negative control.

Alkane monooxygenase originating from *Marinobacter* sp. (UniProt SEQ ID A0A7G2RNR8) was also tested in whole-cell biotransformation of 2 mM 3a resulting in 50% ee.

## Claims

1. Method for the preparation of a compound of the formula (I), which is enriched in one enantiomer having formula (I): 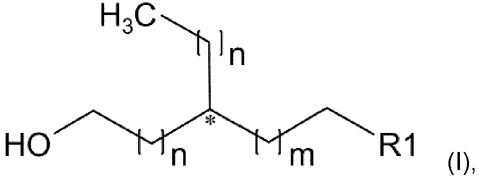 wherein R1 is OH, OCOR2 or COOR3, n is an integer between 0 to 3 and m is an integer between 1 to 10, wherein R2 is H or a C₁ to Cs alkyl group and R3 is H or a C₁ to Cs alkyl group, comprising the step of reacting a compound of formula (II) 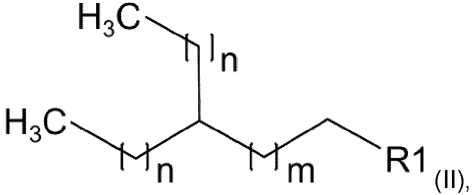 with an alkane monooxygenase.

2. Method according to claim 1, wherein the alkane monooxygenase is an alkane monooxygenase from bacteria, preferably from a genus selected from the group consisting of *Pseudomonas, Marinobacter, Alcanivorax* and *Fontimonas,* more preferably selected from the group consisting of *Pseudomonas putida, Marinobacter sp., Alcanivorax borkumensis* and *Fontimonas thermophila.*

3. Method according to claim 1 or 2, wherein the alkane monooxygenase comprises an amino acid sequence which is at least 80% identical to SEQ ID No. 1, SEQ ID No. 2, SEQ ID No. 3, SEQ ID No. 4, SEQ ID No. 5 or SEQ ID No. 6 or which is encoded by a nucleic acid sequence which is at least 80% identical to SEQ ID No. 7, SEQ ID No. 8, SEQ ID No. 9, SEQ ID No. 10, SEQ ID No. 11 or SEQ ID No. 12

4. Method according to claim 3, wherein the alkane monooxygenase comprising the amino acid sequence SEQ ID No. 1 comprises a mutation at position F164 and/or I233.

5. Method according to any one of claims 1 to 4, wherein the C₁ to Cs alkyl group of R2 and/or R3 is a methyl or ethyl group.

6. Method according to any one of claims 1 to 5, wherein the compound of formula (II) is reacted with the alkane monooxygenase in the presence of an electron transfer protein and an enzyme regenerating the electron transfer protein.

7. Method according to claim 6, wherein the electron transfer protein is rubredoxin and the enzyme regenerating the electron transfer protein is rubredoxin reductase.

8. Method according to claim 7, wherein rubredoxin comprises an amino acid sequence which is at least 80% identical to SEQ ID No. 13 or SEQ ID No. 14 or which is encoded by a nucleic acid sequence which is at least 80% identical to SEQ ID No. 15 or SEQ ID No. 16.

9. Method according to claim 7, wherein rubredoxin reductase comprises an amino acid sequence which is at least 80% identical to SEQ ID No. 17 or which is encoded by a nucleic acid sequence which is at least 80% identical to SEQ ID No. 18

10. Method according to any one of claims 1 to 9, wherein the alkane monooxygenase is an isolated alkane monooxygenase or comprised in a cell expressing the alkane monooxygenase and optionally the electron transfer protein and/or the electron transfer protein reductase, preferably rubredoxin and/or the rubredoxin reductase, or a lysate of said cell.
